# EUROPEAN PATENT APPLICATION

(11) **EP 4 275 589 A1**
(43) Date of publication of application: **15.11.2023**
(21) Application number: 22172793.6
(22) Date of filing: 11.05.2022
(51) Int. Cl.: A61B 5/00, A61B 5/145

(54) **INSERTION SYSTEM**

(71) Applicant: Roche Diabetes Care GmbH, 68305 Mannheim (DE); F. Hoffmann-La Roche AG, 4070 Basel (CH)
(72) Inventor: Braun, Harald, D-68305 Mannheim (DE); Kreutz, Friedhelm, D-68305 Mannheim (DE); Roscher, Olaf, D-68305 Mannheim (DE); Wiegand, Roland Hans, D-68305 Mannheim (DE)
(74) Representative: Altmann Stößel Dick Patentanwälte PartG mbB

(57) **Abstract**

An insertion system (114) comprising at least one screw coupling (118) configured for coupling at least one first element (110) and at least one second element (112) of the insertion system (114) is disclosed. The screw coupling (118) is configured such that the first element (110) and the second element (112) are assembled without rotational movement. The screw coupling (118) comprises
- at least one radially flexible part (120) configured for radial movement and/or deformation when assembling the first element (110) and the second element (112), wherein the radially flexible part (120) is a part of the first element (110) or second element (112);
- elastic means (122) configured for applying a force in an axial direction (124) to the screw coupling (118), wherein the elastic means (122) are preloadable in the axial direction (124) during assembly of the insertion system (114).

## Description

### Technical Field

The invention relates to an insertion system, a kit and a method for assembling a first element and a second element of an insertion system. The devices and the method may be applied in the field of continuous monitoring of the analyte in the body fluid of the user, specifically in the field of home care and in the field of professional care, such as in hospitals. Other applications, however, are also feasible.

### Background art

Monitoring certain body functions, more particularly monitoring one or more analyte concentrations such as one or more metabolite concentration in a body fluid of a user plays an important role in the prevention and treatment of various diseases. Such analytes can include by way of example, but not exclusively, glucose, lactate, cholesterol or other types of analytes and metabolites. Without restricting further possible applications, the invention will be described in the following text with reference to glucose monitoring. However, additionally or alternatively, the invention can also be applied to other types of analytes, such as the analytes mentioned above.

Generally, systems for long-term monitoring of an analyte in a body tissue of a user as well as corresponding insertion devices are known. For example, US9615779B2 describes systems and methods for measuring an analyte in a host. Described are sensor applicators and methods of use with pushbutton activation that implant the sensor, withdraw the insertion needle, engage the transmitter with the housing, and disengage the applicator from the housing, all in one smooth motion.

For coupling of elements of the insertion system usually, a screw coupling is used. Generally, two options are known to the skilled person to mount a screw coupling without play. Firstly, it may be possible to screw on a thread until it reaches a stop, which prevents axial play due to tensioning of the two elements involved. Manufacturing tolerances of the thread may be compensated by an inaccuracy in a rotational orientation of the two elements. In addition, it may be possible to design at one of the two elements that the thread runs out, tapers or changes the slope so that the mating element remains clamped. Even in this case play in axial direction may result in an inaccuracy in a rotational orientation of the two elements. A second option may use pressing over the thread. Even in this case, a certain axial play may be necessary for snapping into the final position. The play has to be designed depending on the slope and/or depth of the thread, but is always greater zero. Thus, however, the two named options may have the disadvantage that either there is an axial play at a defined orientation or, if no play is allowed, that there is an inaccuracy in rotation orientation. In case the connection, e.g. due to technical constrains, has to keep a predefined rotative orientation, there may be the problem that there is a play in axial direction. Said play can only be reduced by using addition elements, which reduces the play once the rotative orientation is reached.

On other technical fields, screw couplings are used, e.g. as described in US 1,0371,870 B2 or DE 10 2016 208367 A1.

Despite the advantage achieved by the above-mentioned devices, several technical challenges remain. For example, improving precision of screw coupling of elements of an insertion system to avoid thread lash as well as imprecision of the relative rotational orientation of the two screw coupling parts is still an unsolved problem.

### Problem to be solved

It is therefore desirable to provide an insertion system, a kit and a method which at least partially address the above-mentioned technical challenges. Specifically, a screw coupling for an insertion system is desirable which allows achieving a defined rotational and axial position of the screw coupling at the same time.

### Summary

This problem is addressed by an insertion system, a kit and a method with the features of the independent claims. Advantageous embodiments which might be realized in an isolated fashion or in any arbitrary combinations are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention, an insertion system is disclosed.

The term "system" as used herein is a broad term and is to be given its ordinary and customary meaning to a person of ordinary skill in the art and is not to be limited to a special or customized meaning. The term specifically may refer, without limitation, to a group of elements which may interact with each other in order to fulfill at least one common function. The at least two elements may be handled independently or may be coupled, connectable or integrateable in order to form a common device. The term "insertion system" as used herein is a broad term and is to be given its ordinary and customary meaning to a person of ordinary skill in the art and is not to be limited to a special or customized meaning. The term specifically may refer, without limitation, to a group elements which are capable of interacting with each other in order to perform at least one transcutaneous or subcutaneous insertion of at least one of the elements into a body tissue of a user, such as by performing an incision or a puncture in a skin of the user and by transferring the at least one of the elements fully or partially into the body tissue. The insertion system may be configured for transcutaneously or subcutaneously inserting a medical device into the body tissue, such as by performing an incision or a puncture in a skin of the user and by transferring the medical device fully or partially into the body tissue.

The term "medical device" as used herein is a broad term and is to be given its ordinary and customary meaning to a person of ordinary skill in the art and is not to be limited to a special or customized meaning. The term specifically may refer, without limitation, to an arbitrary element or article being configured for use in the field of medical technology, specifically in the field of medical analytics or medical diagnostics. The medical device may be configured for performing at least one medical function and/or for being used in at least one medical process, such as one or more of a therapeutic process, a diagnostic process or another medical process.

For example, the medical device may be or may comprise at least one analyte sensor for detecting at least one analyte in a bodily fluid of a user, such as in a bodily fluid contained in a body tissue of the user. The analyte sensor may be configured for being used in qualitatively and/or quantitatively detecting the at least one analyte. The term "analyte" as used herein is a broad term and is to be given its ordinary and customary meaning to a person of ordinary skill in the art and is not to be limited to a special or customized meaning. The term specifically may refer, without limitation, to a chemical and/or biological substance which takes part in the metabolism of the body of the user. Specifically, the analyte may be a metabolite or a combination of two or more metabolites. As an example, the analyte may be selected from the group consisting of: glucose, lactate, triglycerides, cholesterol. Still, other analytes or combinations of two or more analytes may be detected. The body tissue specifically may be or may comprise fatty tissue and/or interstitium. Other types of body tissue, however, are feasible.

The term "analyte sensor" as used herein is a broad term and is to be given its ordinary and customary meaning to a person of ordinary skill in the art and is not to be limited to a special or customized meaning. The term specifically may refer, without limitation, to a sensor which is capable of qualitatively or quantitatively detecting the presence and/or the concentration of the at least one analyte.

The analyte sensor may be an electrochemical analyte sensor. The analyte sensor may comprise at least two electrodes. Specifically, the analyte sensor may comprise at least one two-electrode sensor. The two-electrode sensor may comprise precisely two electrodes, such as a working electrode and at least one further electrode such as a counter electrode, e.g. a working electrode and a combined counter/reference electrode. The working electrode may comprise a working electrode pad and, optionally, at least one test chemical disposed thereon. The counter electrode may comprise a counter electrode pad. Additionally and optionally, one or more redox materials may be disposed thereon. The analyte sensor may further comprise one or more leads for electrically contacting the electrodes. The leads may, during insertion or at a later point in time, be connected to one or more electronic components. For example, the leads may already be connected to the electronic components before insertion of the analyte sensor. For example, the analyte sensor may be a needle-shaped or a strip-shaped analyte sensor having a flexible substrate and the electrodes disposed thereon. As an example, the analyte sensor may have a total length of 5 mm to 50 mm, specifically a total length of 7 mm to 30 mm. The term "total length" within the context of the present invention relates to the overall length of the analyte sensor which means a portion of the analyte sensor which is inserted and the portion of the analyte sensor which may stay outside of the body tissue. The portion of the analyte sensor which is inserted is also called the in-vivo portion, the portion of the analyte sensor which may stay outside of the body tissue is also called the ex vivo portion. For example, the in vivo portion has a length in the range from 3 mm to 12 mm. The analyte sensor may further comprise a biocompatible cover, such as a biocompatible membrane which fully or partially covers the analyte sensor and which prevents the test chemical from migrating into the body tissue and which allows for a diffusion of the body fluid and/or the analyte to the electrodes. Other embodiments of electrochemical analyte sensors, such as three-electrode sensors, may be feasible. For example, the three-electrode sensor may comprise, in addition to the working electrode and the counter electrode, a reference electrode.

The analyte sensor may be an optical analyte sensor. For example, the analyte sensor may comprise a flexible light guide with glucose sensitive coating at its end and/or a tube like carrier with functional elements at inner or outer walls. Other embodiments of the analyte sensor may be possible too.

For example, the medical device may be or may comprise at least one infusion cannula. The term "infusion cannula" as used herein is a broad term and is to be given its ordinary and customary meaning to a person of ordinary skill in the art and is not to be limited to a special or customized meaning. The term specifically may refer, without limitation, to a hollow tube configured for delivering and/or infusing a medication into the body tissue of the user, e.g. for delivering and/or infusing insulin into the body tissue of the user.

The medical device may comprise at least one component which may be configured to stay outside of the body tissue. Further, the medical device may comprise at least one insertable portion. The insertable portion may be configured for being inserted into the body tissue of the user. Specifically, the insertion system may be configured for inserting the insertable portion of the medical device into the body tissue of the user. The medical device, specifically the insertable portion of the medical device, may comprise at least one device selected from the group consisting of: an analyte sensor for detecting at least one analyte in a body fluid of the user, an infusion cannula and stimulating electrodes. Other embodiments may be feasible.

The medical device may be configured to be mounted on a skin site of an extremity of the user. The extremity may be selected from the group consisting of: an arm, specifically an upper arm; a stomach; a shoulder; a back; hip; a leg. Specifically, the extremity may be the upper arm. However, also other applications may be feasible. The term "user" as used herein is a broad term and is to be given its ordinary and customary meaning to a person of ordinary skill in the art and is not to be limited to a special or customized meaning. The term specifically relates to a person intending to monitor an analyte value, such as a glucose value, in the person's body tissue and/or to deliver medication, such as insulin, into the person's body tissue. In an embodiment, the term specifically may refer, without limitation, to a person using the insertion device. However, in an embodiment, the person using the insertion device is different from the user. For example, the medical device may be inserted by a person different from the user into the user's body tissue. For example, the user may be a patient suffering from a disease, such as diabetes.

The term "inserting" as used herein is a broad term and is to be given its ordinary and customary meaning to a person of ordinary skill in the art and is not to be limited to a special or customized meaning. The term specifically may refer, without limitation, to an action or process of one or more of transcutaneously or subcutaneously implanting and/or positioning the medical device into the body tissue of the user. The medical device, such as the analyte sensor, may fully or partially be inserted into the body tissue. The insertion of the medical device may be performed by using the insertion system. After insertion, the medical device or at least a part of the medical device may remain in the body tissue of the user for a predetermined period of time, such as for several hours, specifically for one or more days, more specifically for up to one week, even more specifically for up to two weeks or even more. The medical device may be configured for continuously monitoring and/or detecting the analyte in the body fluid of the user.

The insertion system may comprise at least one insertion component configured for inserting the medical device into the body tissue of the user. The term "insertion component" as used herein is a broad term and is to be given its ordinary and customary meaning to a person of ordinary skill in the art and is not to be limited to a special or customized meaning. The term specifically may refer, without limitation, to an arbitrary element which may be insertable at least partially into the body tissue, particularly in order to deliver or to transfer a further element. The insertion component may be configured for supporting the insertion of the medical device. Specifically, the insertion component may be configured for supporting the insertion of the insertable portion of the medical device. The insertion component may comprise a tip or a sharp end for inserting the medical device, specifically the insertable portion of the medical device, into the body tissue. The insertion component may be or may comprise an insertion cannula or an insertion needle. In the context of the present invention, the insertion component is considered as part of the insertion device. However, in particular during manufacturing of the insertion system, the insertion component may also be considered as a part of the medical device. The term "insertion cannula" as used herein is a broad term and is to be given its ordinary and customary meaning to a person of ordinary skill in the art and is not to be limited to a special or customized meaning. The term specifically may refer, without limitation, to a hollow needle which may be at least partially slotted. The medical device may be received within the insertion cannula, such as within a lumen of the insertion cannula. The term "insertion needle" as used herein is a broad term and is to be given its ordinary and customary meaning to a person of ordinary skill in the art and is not to be limited to a special or customized meaning. The term specifically may refer, without limitation, to a compact needle, specifically without a slot and without any hollow parts. The medical device may be received on an outer surface of the insertion needle.

After insertion, the medical device, specifically the insertable portion of the medical device, may remain at least partially in the body tissue of the user. The insertion component, however, may be retracted from the body tissue of the user into the insertion device after inserting the medical device. For retracting the insertion component, the insertion system may comprise at least one insertion component retractor. The term "insertion component retractor" as used herein is a broad term and is to be given its ordinary and customary meaning to a person of ordinary skill in the art and is not to be limited to a special or customized meaning. The term specifically may refer, without limitation, to at least one element of the insertion system configured for retracting the insertion component. The insertion component retractor may be configured for suspending the insertion component during an insertion movement and pull it out from the skin of the user during a retraction movement. An engagement between the insertion component retractor and the insertion component may be loose, specifically not fastened. The engagement between the insertion component retractor and the insertion component may be established during a production process. The insertion component retractor may comprise at least one finger, gripper, hook, pincer or the like configured for retracting the insertion component. The finger, gripper, hook, pincer or the like may be arranged at a proximal end of the insertion component retractor, wherein, when the insertion device is in use, the proximal end of the insertion component retractor may point towards the body tissue of the user. For example, the insertion component retractor may comprise two or more fingers, grippers, hooks or pincers arranged symmetrically around the insertion component. For example, the insertion device may comprise at least one plunger or pull rod connected to the insertion component. The insertion component retractor may be connected to the plunger or push rod and/or may be configured for grabbing the plunger or pull rod in order to drive the insertion component to perform the retraction movement.

The insertion system may further comprise at least one guide sleeve and at least one insertion sleeve guided therein. The term "guide sleeve" as used herein is a broad term and is to be given its ordinary and customary meaning to a person of ordinary skill in the art and is not to be limited to a special or customized meaning. The term specifically may refer, without limitation, to an enclosure of one or more components configured for guiding at least one movement of components enclosed by the guide sleeve, specifically of the insertion sleeve and the insertion component retractor. The guide sleeve may fully or partially enclose the insertion component retractor, the insertion component and the insertion sleeve. The guide sleeve may be partially enclosed by a housing of the insertion system. The guide sleeve may be essentially rotationally symmetric, specifically in accordance with the symmetry of the housing of the insertion system. For example, in case the housing may have an axial rotational symmetry about an axis such as a cylinder axis or axis of extension, the guide sleeve may have a similar axial rotational symmetry. The guide sleeve may be movable with respect to the housing of the insertion system. For example, when using the insertion system, the guide sleeve may be configured for sliding into the cap of the insertion system. The guide sleeve, in particular a proximal end of the guide sleeve, may be in contact with the user's skin when the insertion system is used. The term "insertion sleeve" as used herein is a broad term and is to be given its ordinary and customary meaning to a person of ordinary skill in the art and is not to be limited to a special or customized meaning. The term specifically may refer, without limitation, to an element configured for at least partially enclosing the insertion component retractor. The insertion sleeve itself may be enclosed fully or partially by the guide sleeve and/or by the housing of the insertion system. The insertion sleeve may be essentially rotationally symmetric, specifically in accordance with the symmetry of the housing and the guide sleeve. For example, the housing and the guide sleeve may have an axial rotational symmetry about an axis such as a cylinder axis or axis of extension, the insertion sleeve may have a similar axial rotational symmetry. Interlocking and/or connection of the elements of the insertion system such as of one or more of guide sleeve, insertion sleeve, insertion component retractor, insertion component may be performed by using one or more of hooks, rods, plungers, wings, grooves, gripper, finger and the like.

As outlined above, the insertion system may comprise a housing. The term "housing" as used herein is a broad term and is to be given its ordinary and customary meaning to a person of ordinary skill in the art and is not to be limited to a special or customized meaning. The term specifically may refer, without limitation, to an element or component having at least one interior space and at least one wall fully or partially surrounding the at least one interior space and providing protection to the interior space, such as one or more of a mechanical protection and a protection against environmental influences such as one or more of moisture, oxygen and microbial contaminations. The housing may generally be adapted to fully or partially surround and/or receive one or more elements in order to provide one or more of a mechanical protection, a mechanical stability, an environmental protection against moisture and/or ambient atmosphere, a shielding against electromagnetic influences or the like. The housing may also provide a basis for attachment and/or holding one or more further components or elements. The housing may comprise an inner structure configured for holding components of the insertion system together, such as at least one latching element. The latching element may interlock the housing with at least one of the other components, such as the guide sleeve and/or the insertion sleeve. The housing may be attached to the insertion sleeve during production. Specifically, the housing may be attached to the insertion sleeve by a snap-fit connection. However, additionally or alternatively, the housing may be attached to the insertion sleeve by gluing, welding, screwing, heat stacking or any other method known to one skilled in the art.

The housing may comprise at least two parts. The housing may comprise an insertion cap. The term "insertion cap" as used herein is a broad term and is to be given its ordinary and customary meaning to a person of ordinary skill in the art and is not to be limited to a special or customized meaning. The term specifically may refer, without limitation, to a part of the housing configured for one or more of triggering, initiating and performing an insertion movement. The insertion cap may be arranged at an upper part of the housing, wherein "upper" may refer to a situation in which the insertion system is oriented in a direction of use. The insertion movement may comprise movement of the insertion cap together with the insertion component retractor and the insertion sleeve from its initial position to its insertion position. The insertion movement may be initiable by an action of the user. Specifically, the action of the user may be or may comprise application of a force to the insertion cap by the user such as by manual pressing and/or pushing the insertion cap downward, specifically in direction to the user's skin. The insertion system may be a mechanical insertion device which may be operated by hand, preferably without the need of electrical or electromechanical actuators. However, other embodiments are feasible.

The housing further may comprise a protective cap. The protective cap may be arranged at a lower part of the housing, wherein "lower" may refer to a situation in which the insertion system is oriented in a direction of use. The term "protective cap" as used herein is a broad term and is to be given its ordinary and customary meaning to a person of ordinary skill in the art and is not to be limited to a special or customized meaning. The term specifically may refer, without limitation, to a part of the housing configured for protecting the medical device and/or the insertion component before use such as during storage, supply, sale and the like. The protective cap may be configured for providing an originality closure of the insertion system. The protective cap may be detachable from the insertion system by at least one twist movement. The detaching of the protective cap may comprise at least partially exposure of the guide sleeve and the insertion component for use of the insertion system.

The insertion system comprises at least one screw coupling configured for coupling at least one first element and at least one second element of the insertion system. The screw coupling is configured such that the first element and the second element are assembled without rotational movement. The screw coupling comprises
- at least one radially flexible part configured for radial movement and/or deformation when assembling the first element and the second element, wherein the radially flexible part is a part of the first or second element;
- elastic means configured for applying a force in an axial direction to the screw coupling, wherein the elastic means are preloadable in the axial direction during assembly of the insertion system.

The term "first element" and the term "second element" as used herein is a broad term and is to be given its ordinary and customary meaning to a person of ordinary skill in the art and is not to be limited to a special or customized meaning. The term specifically may refer, without limitation, to two components of the insertion system. The terms "first" and "second" element, are used purely as names and in particular do not give any information about an order and/or about whether, for example, other elements are present.

The term "coupling" as used herein is a broad term and is to be given its ordinary and customary meaning to a person of ordinary skill in the art and is not to be limited to a special or customized meaning. The term specifically may refer, without limitation, to a mechanical connection of two or more elements. The term "screw coupling" as used herein is a broad term and is to be given its ordinary and customary meaning to a person of ordinary skill in the art and is not to be limited to a special or customized meaning. The term specifically may refer, without limitation, to a mechanical connection of two or more elements using at least one thread. The term "mechanical connection" as used herein is a broad term and is to be given its ordinary and customary meaning to a person of ordinary skill in the art and is not to be limited to a special or customized meaning. The term specifically may refer, without limitation, to a connection of the two or more components by mechanical holding forces. As an example, the mechanical connection may be or may comprise at least one of a form-fit and/or a force-fit connection.

The term "thread", also denoted as screw thread, as used herein is a broad term and is to be given its ordinary and customary meaning to a person of ordinary skill in the art and is not to be limited to a special or customized meaning. The term specifically may refer, without limitation, to at least one structure comprising at least indentation that runs e.g. at least in one or more sections continuously, such as helically, in a cylindrical inner or outer wall.

Each of the first element and the second element may comprise a thread. The first element and the second element may form a matched pair of threads, such as of external and internal threads. The pair of threads of the first element and the second element may be denoted as mating threads. One of the threads of the first element and the second element may be a male thread, wherein the other one may be a female thread. The term "male" thread as used herein is a broad term and is to be given its ordinary and customary meaning to a person of ordinary skill in the art and is not to be limited to a special or customized meaning. The term specifically may refer, without limitation, to a thread on an external surface. The term "female" thread as used herein is a broad term and is to be given its ordinary and customary meaning to a person of ordinary skill in the art and is not to be limited to a special or customized meaning. The term specifically may refer, without limitation, to a thread on an internal surface.

The screw coupling, e.g. one of the threads of the first and the second element, may comprises at least one stop configured for at least partially blocking relative axial movement of the first element and the second element. The term "stop" as used herein is a broad term and is to be given its ordinary and customary meaning to a person of ordinary skill in the art and is not to be limited to a special or customized meaning. The term specifically may refer, without limitation, to a defined end point of a path of motion of the relative movement of the first and second elements. The stop may be formed by an arbitrary shaped element, such as a physical obstacle. The stop may be a component of one or both of the first and second elements or of a further element of the insertion system. At the stop the first element may hit the second element and/or one of the first and second elements may hit the further element of the insertion system. The term "at least partially blocking" as used herein is a broad term and is to be given its ordinary and customary meaning to a person of ordinary skill in the art and is not to be limited to a special or customized meaning. The term specifically may refer, without limitation, to blocking relative axial movement of the first element and the second element such that an axial play is still present.

The screw coupling is configured such that the first element and the second element are assembled without rotational movement. The term "assembled" as used herein is a broad term and is to be given its ordinary and customary meaning to a person of ordinary skill in the art and is not to be limited to a special or customized meaning. The term specifically may refer, without limitation, to a connected state, also referred to as mated. The term "rotational movement" as used herein is a broad term and is to be given its ordinary and customary meaning to a person of ordinary skill in the art and is not to be limited to a special or customized meaning. The term specifically may refer, without limitation, to movement along at least one axis of rotation. The first and the second element may have at least two degrees of freedom with respect to movement relative to each other, such as axial movement and rotational movement. An axial direction may be defined by an axis of extension of the insertion system, e.g. of the first element and the second element. A movement in axial direction may be denoted as axial movement, also denoted as translation. A movement changing radial orientation around the axis of rotation may be denoted as rotational movement or twist. The axis of rotation may be for example, the axis extension. The term "without rotational movement" as used herein is a broad term and is to be given its ordinary and customary meaning to a person of ordinary skill in the art and is not to be limited to a special or customized meaning. The term specifically may refer, without limitation, to the fact that the first element and the second element are assembled by axial movement only, such as without a twisting the first element and the second element relative to each other. The first element and the second element may be assembled by a stricktly axial movement.

The screw coupling may be configured for coupling the first element and the second element by pressing one of the first element and the second element over the other one of the first element and the second element. The term "pressing over" as used herein is a broad term and is to be given its ordinary and customary meaning to a person of ordinary skill in the art and is not to be limited to a special or customized meaning. The term specifically may refer, without limitation, to application of a force in an axial direction such that one of the first element and the second element is sliding over the other one. After pressing one of the first element and the second element over the other, during assembly, an axial play may be present such that the first element and the second element can move relative to each other in the axial direction. The axial play may be e.g. few tenths of a millimeter.

As outlined above, the screw coupling comprises the at least one radially flexible part configured for radial movement and/or deformation when assembling the first element and the second element. The term "flexible" as used herein is a broad term and is to be given its ordinary and customary meaning to a person of ordinary skill in the art and is not to be limited to a special or customized meaning. The term specifically may refer, without limitation, to an elastic property. The term "radially flexible" as used herein is a broad term and is to be given its ordinary and customary meaning to a person of ordinary skill in the art and is not to be limited to a special or customized meaning. The term specifically may refer, without limitation, to the fact that the radially flexible part is configured for motion and/or deformation at a right angle to an axis of rotation. The radially flexible part is a part of the first element or second element. The radially flexible part may be configured for retreating such as by motion and/or deformation in a direction a radial force exerted by the one of the first and second element not comprising the radially flexible part. The retreating may be at least in part in accordance with Hooke's law. For example, the radially flexible part may be configured for radially moving inward when pressed inwards. For example, the radially flexible part may be configured for radially moving outwards when pressed outwards. The flexible part may comprise lower stiffness, e.g. due to its geometry and/or material property, than the surrounding components.

The first element and the second element may be interlocked via grooves and protrusions of the mating threads of the first and second element. For example, one of the first element and the second element may comprise the radially flexible part. The other one of the first element and the second element may comprise the at least one groove for interlocking with at least one element of the radially flexible part and at least one protrusion element configured for applying a radial force on the radially flexible part when coupling the first element and the second element. The groove and protrusion element may be elements of the mating threads. The groove and the protrusion element may be formed by the indentation that runs at least in one or more sections continuously, such as helically, in a cylindrical inner or outer wall. The radially flexible part may be configured for radial movement and/or deformation when pressing over the threads of the first element and the second element. The radially flexible part may comprise at least one inclined surface configured for sliding over the protrusion element. The inclined surface may support sliding and minimizing the force necessary for pressing over. For example, the screw coupling may comprise a snap closure. The term "snap closure" as used herein is a broad term and is to be given its ordinary and customary meaning to a person of ordinary skill in the art and is not to be limited to a special or customized meaning. The term specifically may refer, without limitation, to an arbitrary closure configured for engaging via a snapping or clip mechanism.

The screw coupling may be configured for detachably coupling the first element and the second element. The term "detachably coupling" as used herein is a broad term and is to be given its ordinary and customary meaning to a person of ordinary skill in the art and is not to be limited to a special or customized meaning. The term specifically may refer, without limitation, to the fact that the first and second element can be brought back from a mated state into a disconnected state. The screw coupling can be closed and released at will. For example, the screw coupling may be releasable without using any tools, by manual action. For example, the first element and the second element may be disconnected by twisting one of the first element and the second element. The screw coupling may be detachable only under deformation and/or destruction of at least one of the first element and the second element. The screw coupling may be configured for providing an originality closure of the insertion system.

As outlined above, the screw coupling further comprises elastic means configured for applying a force in the axial direction to the screw coupling. The term "elastic means" as used herein is a broad term and is to be given its ordinary and customary meaning to a person of ordinary skill in the art and is not to be limited to a special or customized meaning. The term specifically may refer, without limitation, to one or more elements having elastic properties and/or a combination of one or more elements configured for providing an elastic function. The elastic means may have linear elastic properties according to Hooke's law. For example, the elastic means may comprise at least one spring element, such as a coil spring or leaf spring, and/or at least one elastic arm, such as an elastic plastic arm.

The term "applying a force in an axial direction" as used herein is a broad term and is to be given its ordinary and customary meaning to a person of ordinary skill in the art and is not to be limited to a special or customized meaning. The term specifically may refer, without limitation, to at least one process of clamping and/or tensioning the first element and the second element against each other. The force in the axial direction may act on the first element and the second element such that both parts are clamped and/or tensioned against each other. For example, as will be outlined in more detail below, the elastic means may be preloadable in the axial direction during assembly of the insertion system. For example, the elastic means may comprise at least one spring element and/or at least one elastic arm and the spring element and/or at least one elastic arm may be preloaded by compression or extension to a distance from its respective equilibrium length. By releasing the load on the elastic means, the elastic means may apply a (opposing) force in the axial direction on the first element and the second element. For example, the elastic means may push back to its equilibrium length, e.g. in accordance to Hooke's law in case of linear elastic properties. In this case, the force in the axial direction applied by the elastic means may be linearly proportional to the distance from its equilibrium length. The screw coupling may comprise the at least one stop configured for blocking further movement in the axial direction. The spring tension of the elastic means may be configured for preventing (re-)moving in the opposite axial direction. However, other embodiments are feasible. For example, the elastic means may have elastic properties deviating from linear elastic properties. For example, the releasing of the load may comprise releasing the load incompletely, e.g. up to a defined threshold.

The elastic means may be preloadable in the axial direction during assembly of the insertion system. The term "preloadable" as used herein is a broad term and is to be given its ordinary and customary meaning to a person of ordinary skill in the art and is not to be limited to a special or customized meaning. The term specifically may refer, without limitation, to the fact that at least one load is applied to the elastic means as a result of the assembly, e.g. of the coupling of the first element and the second element. The load may be applied directly and/or indirectly to the elastic means. For example, in case of directly applying the load to the elastic means, means to preload may contact the elastic means. For example, in case of indirectly applying the load, the load may be applied to a further element which in turn acts on the elastic means. For example, the elastic means may be deformed, e.g. compressed, in the axial direction during assembly of the insertion system thereby preloading the elastic means. The load for preloading the elastic means may be applied by an external force on the elastic means, e.g. by using means to preload. The term "external force" as used herein is a broad term and is to be given its ordinary and customary meaning to a person of ordinary skill in the art and is not to be limited to a special or customized meaning. The term specifically may refer, without limitation, to a force which at least partially is applied from outside the insertion system. During assembly, the defomation may be generated by applying the external force and may allow coupling of the first element and the second element. The elastic means may be configured for driving at least one axial and relative move of the first element and the second element when the external force on the elastic means is withdrawn such that the first element and the second element are positioned axially and rotative with respect to each other. Thus, after assembly of the first and second element, the force, and thereby the deformation, on the elastic means is reduced thereby removing the axial and radial play between the first element and the second element. The withdrawing of the external force may comprise at least partially reducing the force. For example, the external force may be reduced such that it is still greater zero. The screw coupling may allow to avoid thread lash as well as imprecision of the relative rotational orientation of the first element and the second element. The screw coupling may enable an assembly of e.g. two sleeves, without rotational movement by radial flexibility of at least one part of the screw coupling, therewith gaining a high precision of the relative rotational orientation. After assembly, an axial movement within the screw coupling can be avoided by applying a force in axial direction exerted by the elastic means, removing clearance in axial direction. The screw coupling may achieve a defined rotational and axial position of the screw coupling at the same time.

The housing may comprise at least one opening. For example, the opening may be comprised by the insertion cap. The term "opening" as used herein is a broad term and is to be given its ordinary and customary meaning to a person of ordinary skill in the art and is not to be limited to a special or customized meaning. The term specifically may refer, without limitation, to an element of the housing configured for providing an access from an outside of the insertion system to the elastic means. The elastic means may be preloadable via the opening of the housing. The openings may be configured such that an external force can be applied in an axial direction to the elastic means via means to preload. For example, the means to preload comprise at least one pin. For example, the opening may be a hole. The hole may have a shape matching the shape of the pin, e.g. a circular hole.

For example, the first element is the guide sleeve and the second element is the protective cap of the housing. The elastic means may be coupled to the guide sleeve. The elastic means may be positioned on an inner surface of the insertion cap. The opening may be comprised by the insertion cap. Additionally or alternative, the elastic means may be embodied as part of the protective cap. The opening may be comprised by the protective cap.

In a further aspect of the invention, a kit comprising at least one insertion system according to any one of the embodiments disclosed above and/or according to any one of the embodiments disclosed in further detail below is disclosed. For possible definitions and options, reference may be made to the disclosure of the insertion system according to the present invention. The term "kit" as used herein is a broad term and is to be given its ordinary and customary meaning to a person of ordinary skill in the art and is not to be limited to a special or customized meaning. The term specifically may refer, without limitation, to a combination of at least two items which might as an example, may be supplied conjointly in a package, which may interact in order to fulfill at least one common purpose.

The kit further comprises means to preload the elastic means during assembly. For example, the means to preload comprise at least one pin.

In a further aspect of the invention, a method for assembling a first element and a second element of an insertion system using a kit according to any one of the embodiments disclosed above and/or according to any one of the embodiments disclosed in further detail below is disclosed. Thus, for possible definitions and options, reference may be made to the disclosure of the insertion system and the kit according to the present invention. The method comprises the following steps which specifically may be performed in the given order. The method may comprise further method steps which are not listed.

The method comprises the following steps:
a) applying an external force on the elastic means using the means to preload, wherein the external force acts in an axial direction;
b) assembling by a strictly axial movement the first element and the second element, wherein the assembling comprises interlocking the first element and the second element by using at least one radially flexible part;
c) withdrawing the external force on the elastic means.

Summarizing and without excluding further possible embodiments, the following embodiments may be envisaged:
Embodiment 1. An insertion system comprising at least one screw coupling configured for coupling at least one first element and at least one second element of the insertion system, wherein the screw coupling is configured such that the first element and the second element are assembled without rotational movement, wherein the screw coupling comprises
   - at least one radially flexible part configured for radial movement and/or deformation when assembling the first element and the second element, wherein the radially flexible part is a part of the first element or second element;
   - elastic means configured for applying a force in an axial direction to the screw coupling, wherein the elastic means are preloadable in the axial direction during assembly of the insertion system.
Embodiment 2. The insertion system according to the preceding embodiment, wherein the elastic means comprise at least one spring element and/or at least one elastic arm.
Embodiment 3. The insertion system according to any one of the preceding embodiments, wherein the insertion system comprises a housing, wherein the housing comprises at least one opening, wherein the elastic means are preloadable via the opening of the housing.
Embodiment 4. The insertion system according to the preceding embodiment, wherein the opening is configured such that an external force can be applied in an axial direction to the elastic means via means to preload.
Embodiment 5. The insertion system according to any one of the preceding embodiments, wherein the screw coupling is configured for preventing axial movement of the first element and the second element.
Embodiment 6. The insertion system according to any one of the preceding embodiments, wherein the elastic means is configured for driving at least one axial and relative move of the first element and the second element when an external force on the elastic means is withdrawn such that the first element and the second element are positioned axially and rotative with respect to each other.
Embodiment 7. The insertion system according to any one of the preceding embodiments, wherein the insertion system comprises a housing, wherein the first element is a guide sleeve and the second element is a protective cap of the housing, wherein the housing comprises an insertion cap.
Embodiment 8. The insertion system according to the preceding embodiment, wherein the housing comprises at least one opening, wherein the elastic means are preloadable via the opening, wherein the housing comprises an insertion cap and the opening is comprised by said insertion cap, wherein the elastic means are coupled to the guide sleeve, wherein the elastic means are positioned on an inner surface of the insertion cap.
Embodiment 9. The insertion system according to any one of the two preceding embodiments, wherein the elastic means are embodied as part of the protective cap, wherein the opening is comprised by the protective cap.
Embodiment 10. The insertion system according to any one of the preceding embodiments, wherein the insertion system is configured for inserting at least one medical device into a body tissue of a user, wherein the medical device comprises at least one device selected from the group consisting of an analyte sensor for detecting at least one analyte in a body fluid of a user and an infusion cannula.
Embodiment 11. The insertion system according to any one of the preceding embodiments, wherein each of the first element and the second element comprises a thread, wherein the screw coupling is configured for coupling the first element and the second element by pressing one of the first element and the second element over the other one of the first element and the second element, wherein the radially flexible part is configured for radial movement and/or deformation when pressing over the threads of the first element and the second element.
Embodiment 12. The insertion system according to any one of the preceding embodiments, wherein one of the first element and the second element comprises the radially flexible part, wherein the other one of the first element and the second element comprises at least one groove for interlocking with at least one element of the radially flexible part and at least one protrusion element configured for applying a radial force on the radially flexible part when coupling the first element and the second element.
Embodiment 13. The insertion system according to the preceding embodiment, wherein the radially flexible part comprises at least one inclined surface configured for sliding over the protrusion element.
Embodiment 14. The insertion system according to any one of the preceding embodiments, wherein the screw coupling comprises at least one stop configured for at least partially blocking relative axial movement of the first element and the second element.
Embodiment 15. The insertion system according to any one of the preceding embodiments, wherein the screw coupling is configured for detachably coupling the first element and the second element.
Embodiment 16. A kit comprising at least one insertion system according to any one of the preceding embodiments and means to preload the elastic means during assembly.
Embodiment 17. The kit according to the preceding embodiment, wherein the means to preload comprise at least one pin.
Embodiment 18. A method for assembling a first element and a second element of an insertion system using a kit according to any one of the preceding embodiments, wherein the method comprises the following steps:
   a) applying an external force on the elastic means using the means to preload, wherein the external force acts in an axial direction;
   b) assembling by a strictly axial movement the first element and the second element, wherein the assembling comprises interlocking the first element and the second element by using at least one radially flexible part;
   c) withdrawing the external force on the elastic means.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figures 1A to IE: show a method for assembling a first element and a second element of an insertion system;
- Figure 2: shows an embodiment of an insertion system with coupled first and second elements;
- Figures 3A to 3D: shows a crossectional view of the assembled insertion system of Figure 2 without a protective cap (Fig. 3A); a top view of the insertion sleeve with the insertion component retractor and insertion cap (Fig. 3B); a perspective view of the insertion sleeve with the insertion component retractor and insertion cap (Fig. 3C); a perspective view of the protective cap (Fig. 3D); and
- Figure 4: shows a crossectional view of a detail of the assembled insertion system of Figure 2.

### Detailed description of the embodiments

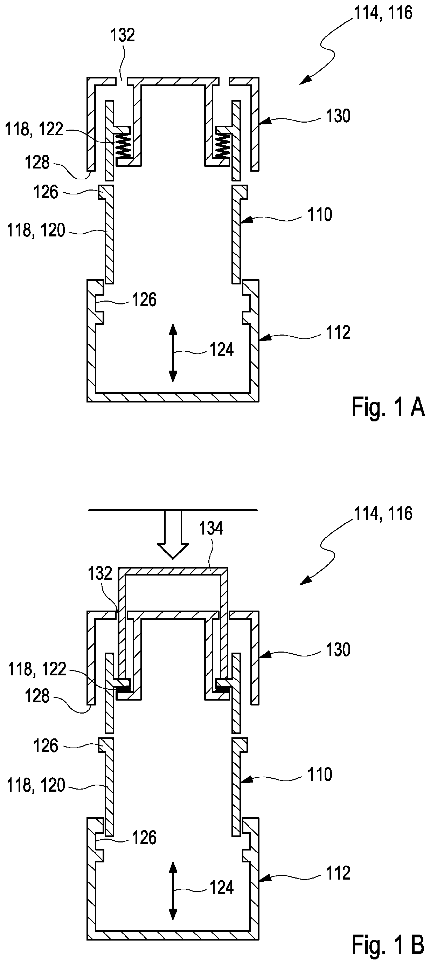

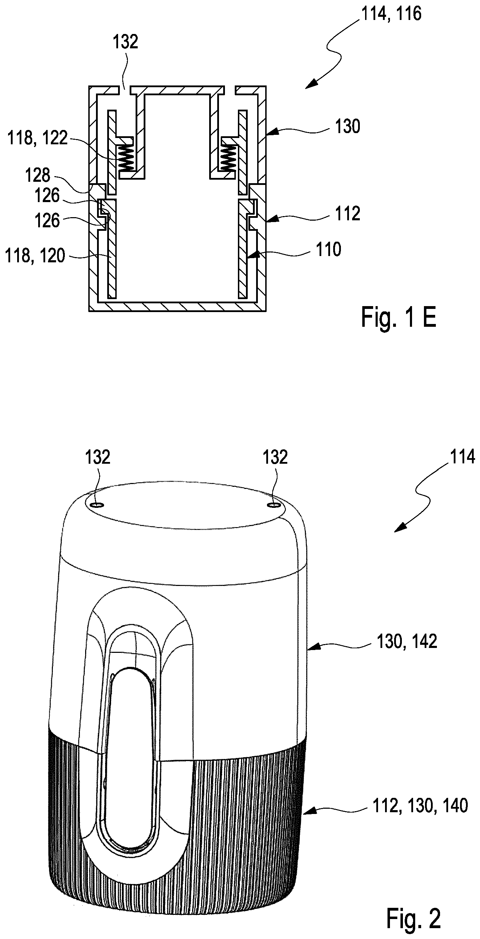

Figures 1A to IE shows an exemplary embodiment of a method for assembling a first element 110 and a second element 112 of an insertion system 114 using a kit 116 according to the present invention.

Figure 1A shows the insertion system 114 before assembling. The insertion system 114 comprises at least one screw coupling 118 configured for coupling the first element 110 and the second element 112.

The screw coupling 118 comprises at least one radially flexible part 120 configured for radial movement and/or deformation when assembling the first element 110 and the second element 112. The radially flexible part is a part of the first element 110 or the second element 112. The screw coupling 118 comprises elastic means 122 configured for applying a force in an axial direction 124 to the screw coupling 118. The elastic means 122 are preloadable in the axial direction 124 during assembly of the insertion system 114.

Each of the first element 110 and the second element 112 may comprise a thread 126. The first element 110 and the second element 112 may form a matched pair of threads 126, such as of external and internal threads. In this embodiment, the first element 110 may be a male thread and the second element 112 may be a female thread.

The elastic means 122 may comprise one or more elements having elastic properties and/or a combination of one or more elements configured for providing an elastic function. The elastic means 122 may have linear elastic properties according to Hooke's law. For example, the elastic means 122 may comprise at least one spring element, such as a coil spring as shown in Figures 1A to IE. Before assembling of the first element 110 and the second element 112 the elastic means 122 may be relaxed having a minimum preload. The elastic means may be arranged between a stop 128 and an inner part of the thread 126 of the first element 110.

The elastic means 122 may be preloadable in the axial direction 124 during assembly of the insertion system 114. Figure 1B shows preloading the elastic means during assembly. Specifically, Figure 1B shows step a) of the method comprising applying an external force on the elastic means 122 using the means to preload 134, wherein the external force acts in an axial direction 124. For example, the elastic means 122 may be deformed, e.g. compressed, in the axial direction 124 during assembly of the insertion system 114 thereby preloading the elastic means 122. The load for preloading the elastic means 122 may be applied by an external force on the elastic means 122, e.g. by using means to preload 134. As shown in Figure 1B, the insertion system 114 may comprise a housing 130. The housing 130 may comprise at least one opening 132. The opening 132 may be configured for providing an access from an outside of the insertion system 114 to the elastic means 122. The elastic means 122 may be preloadable via the opening 132 of the housing 130. The opening 132 may be configured such that an external force can be applied in an axial direction 124 to the elastic means 122 via means to preload 134. For example, the means to preload 134 comprise at least one pin. For example, the opening 132 may be a hole. The hole may have a shape matching the shape of the pin, e.g. a circular hole. In this position, the tension on the elastic means 122 is at its highest. In this state, the first element 110 and the second element 112 can be coupled.

The screw coupling 118 is configured such that the first element 110 and the second element 112 are assembled without rotational movement. The first element 110 and the second element 112 may be assembled by axial movement only, such as without a twisting the first element 110 and the second element 112 relative to each other. The first element 110 and the second element 112 may be assembled by a stricktly axial movement.

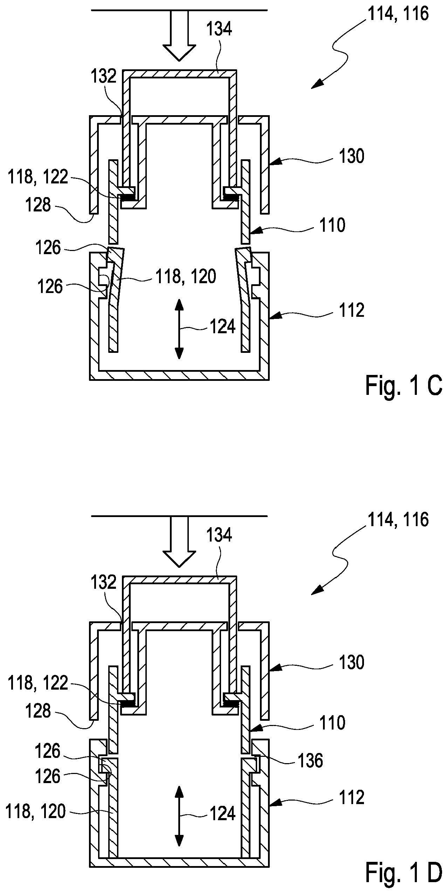

Figure 1C shows the assembling by a strictly axial movement the first element 110 and the second element 112, step b). During preloading the elastic means 122, the second element 112 may be pressed over the first element 110. The pressing over comprises application of a force in an axial direction 124 such that one of the first element 110 and the second element 114 is sliding over the other one. As outlined above, the screw coupling 118 comprises the at least one radially flexible part 120 configured for radial movement and/or deformation when assembling the first element 110 and the second element 112. The radially flexible part 120 may be configured for motion and/or deformation at a right angle to an axis of rotation. The radially flexible part 120 is a part of the first element 110 in this embodiment. The radially flexible part 120 may be configured for retreating such as by motion and/or deformation in a direction a radial force exerted by the second element 112. The retreating may be at least in part in accordance with Hooke's law. For example, the radially flexible part 120 may be configured for radially moving inward when pressed inwards.

As shown in Figure 1D, after pressing over, during assembly, an axial play 136 may be present such that the first element 110 and the second element 112 can move relative to each other in the axial direction 124. The axial play 136 may be e.g. few tenths of a millimeter. The stop 128 may be configured for at least partially blocking relative axial movement of the first element 110 and the second element 122. The stop 128 may be formed by an arbitrary shaped element, such as a physical obstacle. The stop 128 may be a component of one or both of the first element 110 and second element 112.

Figure IE shows the assembled insertion system 114. The elastic means 122 may be configured for driving at least one axial and relative move of the first element 110 and the second element 112 when the external force on the elastic means 122 is withdrawn (step c) such that the first element 110 and the second element 112 are positioned axially and rotative with respect to each other. Thus, after assembly of the first and second element 110, 112, the force, and thereby the deformation, on the elastic means 122 is reduced thereby removing the axial and radial play between the first element 110 and the second element 112. The withdrawing of the external force may comprise at least partially reducing the force. For example, the external force may be reduced such that it is still greater zero. The screw coupling 118 may allow to avoid thread lash as well as imprecision of the relative rotational orientation of the first element 110 and the second element 112. The screw coupling 118 may enable an assembly of e.g. two sleeves, without rotational movement by radial flexibility of at least one part of the screw coupling 118, therewith gaining a high precision of the relative rotational orientation. After assembly, an axial movement within the screw coupling 118 can be avoided by applying a force in axial direction exerted by the elastic means 122, removing clearance in axial direction. The screw coupling 118 may achieve a defined rotational and axial position of the screw coupling 118 at the same time.

The screw coupling 118 may be configured for detachably coupling the first element and the second element. The screw coupling 118 can be closed and released at will. For example, the screw coupling 118 may be releasable without using any tools, by manual action. For example, the first element 110 and the second element 112 may be disconnected by twisting one of the first element 110 and the second element 112. The screw coupling 118 may be detachable only under deformation and/or destruction of at least one of the first element 110 and the second element 112. The screw coupling 118 may be configured for providing an organility closure of the insertion system 114.

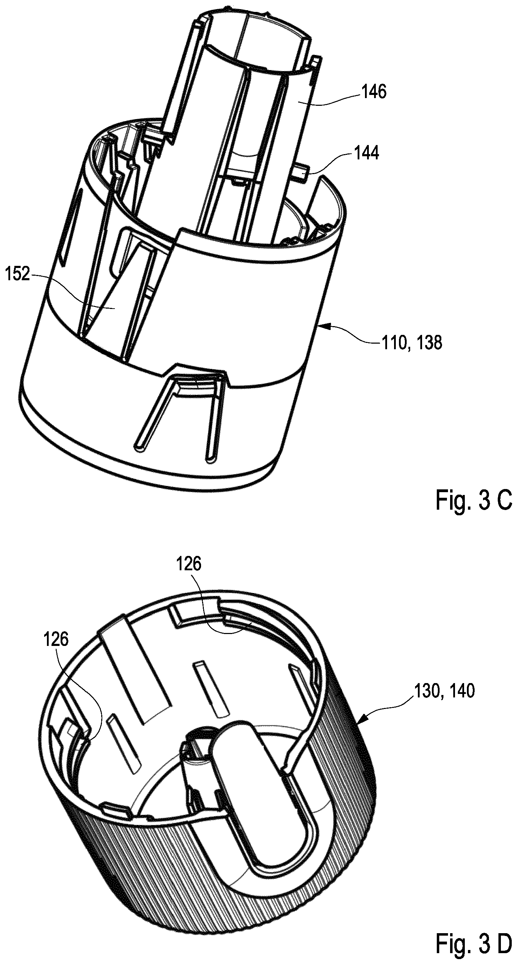

Figure 2 shows an embodiment of an insertion system 114 with coupled first element 110 (not visible here) and second element 112. The second element 112 may be the protective cap 140 of the housing 130. Figure 3D shows a perspective view of the protective cap 140. The housing 130 further comprises an insertion cap 142. The insertion cap 142 may comprise openings 132, e.g. two circular openings as shown in Figure 2.

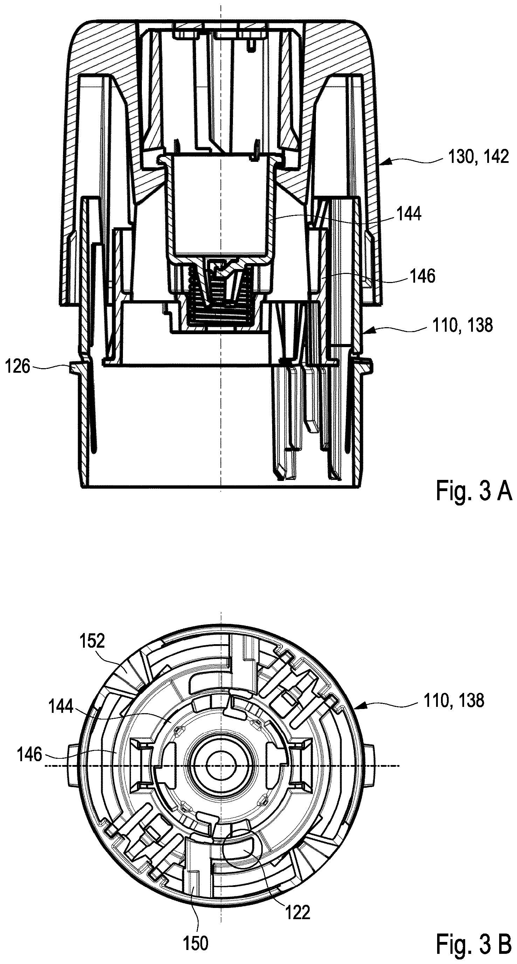

Figure 3A shows a crossectional view of the assembled insertion system 114 of Figure 2 without the protective cap 140. In this embodiment, the first element 110 is a guide sleeve 138. The insertion system 114 may comprise at least one insertion component retractor 144. The insertion system 114 may further comprise at least one insertion sleeve 146 guided in the guide sleeve 138. As shown in Figure 3C, without the protective cap 140, downward movement the insertion sleeve 146 may be limited by wings 152 of the guide sleeve 138, which extend inwardly from the guide sleeve 138 into grooves of the insertion sleeve 146. The insertion system 114 further comprises elastic means 122, e.g. as visible in Figure 3B. The elastic means 122 may be coupled to the guide sleeve 138. At least one part of the elastic means 122 may rest on a part of the insertion sleeve 146, as highlighted with a circle around an arm of the elastic means 122. The elastic means 122 may comprise at least one connecting bar 150. The connecting bar 150 may couple the guide sleeve 138 and the elastic means 122. The arms of the elastic means 122 may be configured for blocking upward movement of the insertion sleeve 146 relative to the guide sleeve 138.

The elastic means 122 may be preloadable via the opening 132 of the housing 130 by using means to preload 134 e.g. at least one pin. The means to preload 134 may be pushed via openings 132 inside the housing 130 until they reach onto the connecting bars 150. During assembly, the elastic means 122 may be preloaded by an external force. The external force may be applied via the means to preload 134 onto the connecting bars 150. Thereby the guide sleeve 138 and the connecting bars 150 move downwards. Due to said downward movement of the guide sleeve 138, the arms of the elastic means 122 may be pushed upwards by the insertion sleeve 146. In this status, the tension of the arms may be at highest.

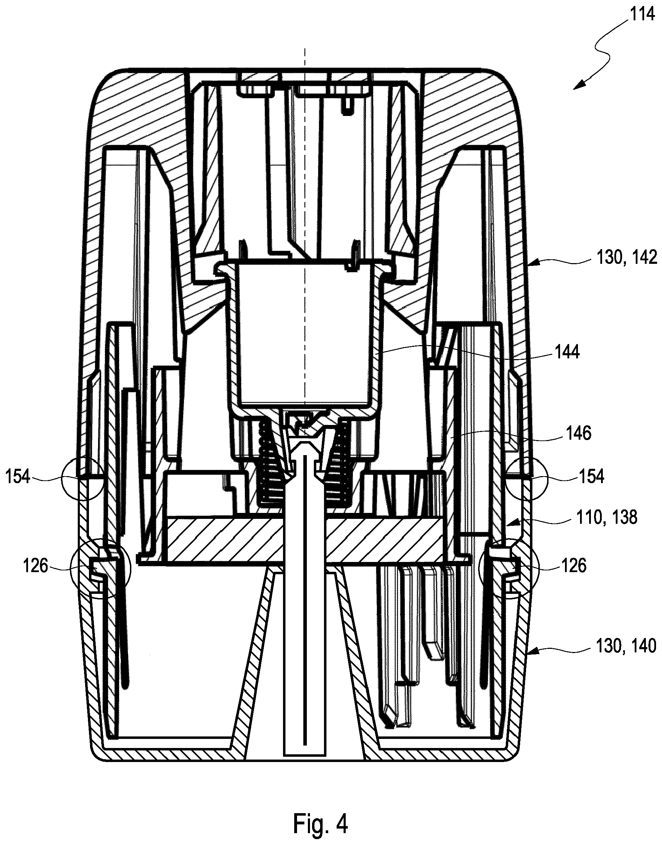

The insertion system 114 may be in this status prepared such that the second element 112 can be assembled, e.g. by pressing one of the guide sleeve 138 and the protective cap 140 over the other one of the guide sleeve 138 and the protective cap 140. When the guide sleeve 138 and the protective cap 140 are assembled into their final position, there may be a play 136 relative to each other. In the assembled status, i.e. with the protective cap 140 as shown in e.g. Figure 4, downward movement of the insertion cap 142 and upwards movement of the protective cap 140 may be limited via a stop 154 (highlighted with a circle in Figure 4) at which the insertion cap 142 and the protective cap 140 hit each other. In addition, the thread 126 is visible in Figure 4, which is formed by protrusions from an outer wall of the guide sleeve 138 and corresponding grooves of an inner wall of the protective cap 140. The thread 126 is highlighted with a circle in Figure 4.

Then the external force may be reduced, e.g. by removing the means to preload 134. The reducing of the external force may trigger that the insertion sleeve 146 is pushed downwards due to the tension of the arms of the elastic means 122 and the guide sleeve 138 and the connecting bars 150 move upwards. Thereby the guide sleeve 138 and the protective cap 140 may be moved relative to each other such that the axial play 136 is compensated. In this position, the first element 110 and the second element 112 are positioned axially and rotative with respect to each other.

### List of reference numbers

- 110: first element
- 112: second element
- 114: insertion system
- 116: kit
- 118: screw coupling
- 120: radially flexible part
- 122: elastic means
- 124: axial direction
- 126: thread
- 128: stop
- 130: housing
- 132: openings
- 134: means to preload
- 136: axial play
- 138: guide sleeve
- 140: protective cap
- 142: insertion cap
- 144: insertion component retractor
- 146: insertion sleeve
- 150: connecting bar
- 152: wing
- 154: stop

## Claims

1. An insertion system (114) comprising at least one screw coupling (118) configured for coupling at least one first element (110) and at least one second element (112) of the insertion system (114), wherein the screw coupling (118) is configured such that the first element (110) and the second element (112) are assembled without rotational movement, wherein the screw coupling (118) comprises
- at least one radially flexible part (120) configured for radial movement and/or deformation when assembling the first element (110) and the second element (112), wherein the radially flexible part (120) is a part of the first element (110) or second element (112);
- elastic means (122) configured for applying a force in an axial direction (124) to the screw coupling (118), wherein the elastic means (122) are preloadable in the axial direction (124) during assembly of the insertion system (114).

2. The insertion system (114) according to the preceding claim, wherein the elastic means (122) comprise at least one spring element and/or at least one elastic arm.

3. The insertion system (114) according to any one of the preceding claims, wherein the insertion system (114) comprises a housing (130), wherein the housing (130) comprises at least one opening (132), wherein the elastic means (122) are preloadable via the opening (132) of the housing (130).

4. The insertion system (114) according to the preceding claim, wherein the opening (132) is configured such that an external force can be applied in an axial direction to the elastic means (122) via means to preload (134).

5. The insertion system (114) according to any one of the preceding claims, wherein the screw coupling (118) is configured for preventing axial movement of the first element (110) and the second element (112).

6. The insertion system (114) according to any one of the preceding claims, wherein the elastic means (122) is configured for driving at least one axial and relative move of the first element (110) and the second element (112) when an external force on the elastic means (122) is withdrawn such that the first element (110) and the second element (112) are positioned axially and rotative with respect to each other.

7. The insertion system (114) according to any one of the preceding claims, wherein the insertion system (114) comprises a housing (130), wherein the first element (110) is a guide sleeve (138) and the second element (112) is a protective cap (140) of the housing (130), wherein the housing (130) further comprises an insertion cap (142).

8. The insertion system (114) according to the preceding claim, wherein the housing (130) comprises at least one opening (132), wherein the elastic means (122) are preloadable via the opening (132), wherein the opening (132) is comprised by said insertion cap (142), wherein the elastic means (122) are coupled to the guide sleeve (138), wherein the elastic means (122) are positioned on an inner surface of the insertion cap (142).

9. The insertion system (114) according to any one of the preceding claims, wherein the insertion system (114) is configured for inserting at least one medical device into a body tissue of a user, wherein the medical device comprises at least one device selected from the group consisting of an analyte sensor for detecting at least one analyte in a body fluid of a user and an infusion cannula.

10. The insertion system (114) according to any one of the preceding claims, wherein each of the first element (110) and the second element (112) comprises a thread (126), wherein the screw coupling (118) is configured for coupling the first element (110) and the second element (112) by pressing one of the first element (110) and the second element (112) over the other one of the first element (110) and the second element (112), wherein the radially flexible part (120) is configured for radial movement and/or deformation when pressing over the threads (126) of the first element (110) and the second element (112).

11. The insertion system (114) according to any one of the preceding claims, wherein one of the first element (110) and the second element (112) comprises the radially flexible part (120), wherein the other one of the first element (110) and the second element (112) comprises at least one groove for interlocking with at least one element of the radially flexible part (120) and at least one protrusion element configured for applying a radial force on the radially flexible part when coupling the first element (110) and the second element (112).

12. The insertion system (114) according to any one of the preceding claims, wherein the screw coupling (118) comprises at least one stop (128) configured for at least partially blocking relative axial movement of the first element (110) and the second element (112).

13. The insertion system (114) according to any one of the preceding claims, wherein the screw coupling (118) is configured for detachably coupling the first element (110) and the second element (112).

14. A kit (116) comprising at least one insertion system (114) according to any one of the preceding claims and means to preload (134) the elastic means (122) during assembly.

15. A method for assembling a first element (110) and a second element (112) of an insertion system (114) using a kit (116) according to the preceding claim, wherein the method comprises the following steps:
a) applying an external force on the elastic means (122) using the means to preload (134), wherein the external force acts in an axial direction;
b)assembling by a strictly axial movement the first element (110) and the second element (112), wherein the assembling comprises interlocking the first element (110) and the second element (112) by using at least one radially flexible part (120);
c) withdrawing the external force on the elastic means (122).
